# EUROPÄISCHE PATENTSCHRIFT

(11) **EP 0 635 475 B1**
(45) Veröffentlichungstag und Bekanntmachung des Hinweises auf die Patenterteilung: **28.05.1997**
(21) Anmeldenummer: 94111132.0
(22) Anmeldetag: 18.07.1994
(51) Int. Cl.: C07C 53/126, C07C 51/305

(54) **Verfahren zur kontinuierlichen Herstellung höhermolekularer Carbonsäuren**
Process for the continuous production of higher molecular carboxylic acids
Procédé pour la préparation continue des acides carboxyliques supérieurs

(30) Priorität: 23.07.1993 DE 4324719
(43) Veröffentlichungstag der Anmeldung: 25.01.1995
(73) Patentinhaber: HOECHST AKTIENGESELLSCHAFT, 65929 Frankfurt am Main (DE)
(72) Erfinder: Heinrichs, Franz-Leo, Dr., D-86456 Gablingen (DE); Hohner, Gerd, Dr., D-86368 Gersthofen (DE); Lukasch, Anton, D-86405 Meitingen (DE); Petz, Karl, Dr., D-86356 Neusäss (DE)

(56) Entgegenhaltungen:
- DE-A- 2 165 858
- DE-C- 2 855 263

## Beschreibung

Die Erfindung bezieht sich auf ein Verfahren zur Herstellung höhermolekularer Carbonsäuren durch Oxidation von ethylenisch ungesättigten Verbindungen mit Chromsäure.

Zur Herstellung von höhermolekularen, linearen aliphatischen Carbonsäuren sind zahlreiche Synthesen bekannt. Technisch genutzt werden aber nur wenige Verfahren.
So wird der Bedarf an höhermolekularen Fett- oder Wachssäuren fast ausschließlich aus Naturprodukten gedeckt.

Technisch realisiert ist die Oxidation von Paraffinen zu Carbonsäuren. Nachteilig bei diesen Verfahren ist aber, daß der Angriff des Oxidationsmittels nicht gesteuert werden kann und eine breite Palette an Carbonsäuren und anderen sauerstoffhaltigen Verbindungen entsteht.

Diese Nachteile können vermieden werden, wenn als Rohstoffe längerkettige Olefine und als Oxidationsmittel beispielsweise schwefelsaure Chromsäure eingesetzt werden. Bekannt sind ein- oder mehrstufige Verfahren zur Oxidation von Olefinen (vgl. DE 2 165 858, DE 2 262 130, DE 23 10 425). Diese Verfahren haben neben der unvollständigen Umsetzung, dem hohen Oxidationsmittelverbrauch und der schlechten Abtrennung noch einen weiteren Nachteil, daß die Dispergierung des Olefins im Oxidationsmittel nur ungenügend erfolgt. Außerdem kommt es zur Bildung einer viskosen, chromhaltigen organischen Phase, die einen geregelten Ablauf der Reaktion stört.

Es ist bekannt, daß die Nachteile einer diskontinuierlichen Arbeitsweise bei kontinuierlicher Arbeitsweise ausgeglichen werden können. So wird beispielsweise die oxidative Bleichung von Naturwachsen zur besseren Handhabung der zähen Reaktionsmischung unter Ausnutzung gasförmiger Oxidationsprodukte in einem Reaktor mit der Charakteristik eines engen Verweilzeitspektrums durchgeführt (vgl. DE 28 55 263).

Es wurde gefunden, daß die Oxidation von Olefinen in einer modifizierten Verfahrensweise möglich ist.

Die Erfindung betrifft somit ein Verfahren zur kontinuierlichen Herstellung von höhermolekularen, linearen aliphatischen Carbonsäuren durch Oxidation von Olefinen mit 16 bis 70 C-Atomen mit ca. 1-molarer, bezogen auf CrO₃, Chromschwefelsäure bei einer Temperatur von 90 bis 200°C und einem Druck von 100 mbar bis 20 bar, dadurch gekennzeichnet, daß die Reaktion im Gleichstrom in mindestens einem Reaktor mit engem Verweilzeitspektrum durchgeführt wird, wobei die Durchmischung der Reaktanden durch den infolge der Reaktionswärme entstehenden Wasserdampf und das Reaktionsgas sowie zusätzliches Einblasen von Luft erfolgt.

Das erfindungsgemäße Verfahren wird in kontinuierlich betriebenen Reaktoren mit der Charakteristik eines engen Verweilzeitspektrums durchgeführt, wie beispielsweise in Rohrreaktoren, Rührkesselkaskaden oder kaskadierten Blasensäulenreaktoren mit Siebböden, wobei die kaskadierten Blasensäulenreaktoren bevorzugt verwendet werden. Diese Reaktoren zeichnen sich vor allem dadurch aus, daß die Durchmischung der Reaktanten durch den infolge der exothermen Reaktion entstehenden Wasserdampf, durch die Freisetzung gasförmiger Reaktionsprodukte, durch das Einblasen von Luft/Wasserdampf und durch die Anordnung und Form der Siebböden sehr effektiv erfolgt, ohne daß dafür der Einbau bewegter mechanischer Teile notwendig ist. Ein weiterer Vorteil der Blasensäulen-Kaskadenreaktoren liegt darin, daß durch den entstehenden Wasserdampf niedermolekulare und wasserdampfflüchtige Oxidationsprodukte aus dem Reaktor ausgetragen werden. Ohne diesen Strippvorgang würden diese Produkte entweder in der verbrauchten Chromschwefelsäure verbleiben und deren Regenerierung stören, oder sie verbleiben im Oxidat und stören bei der weiteren Verwendung dieses Oxidats.

Die für das erfindungsgemäße Verfahren verwendete Apparatur zeigen die Figuren, wobei in Figur 1 eine Apparatur für eine einstufige Arbeitsweise, in Figur 2 eine Apparatur für eine zweistufige Arbeitsweise und in Figur 3 eine Apparatur für eine dreistufige Arbeitsweise dargestellt ist. Es bedeuten in den Figuren
- 111 =: Reaktor für die 1. Stufe
- 112 =: Zuleitung für Olefin
- 113 =: Zuleitung für Chromschwefelsäure
- 114 =: Zuleitung für Luft
- 115 =: Rohrleitung
- 116 =: Zuleitung für Trennmittel
- 211 =: Reaktor für die 2. Stufe
- 212 =: Zuleitung für Olefin/Carbonsäuregemisch
- 213 =: Zuleitung für frische Chromschwefelsäure
- 214 =: Zuleitung für Luft
- 215 =: Rohrleitung
- 216 =: Trenngefäß
- 217 =: Ableitung
- 311 =: Reaktor für die 3. Stufe
- 312 =: Zuleitung für Olefin/Carbonsäuregemisch
- 313 =: Zuleitung für frische Chromschwefelsäure
- 314 =: Zuleitung für Luft
- 315 =: Rohrleitung
- 316 =: Trenngefäß
- 317 =: Ableitung
- 411 =: Entgasungsbehälter
- 412 =: Trenngefäß für Endprodukt
- 413 =: Entchromer
- 414 =: Kondensatabscheider
- 415 =: Rohrleitung
- 416 =: Rohrleitung
- 417 =: Ableitung
- 418 =: Abgasleitung
- 419 =: Abgasleitung
- 420 =: Abgasleitung
- 421 =: Ableitung
- 422 =: Austragsleitung für Endprodukt
- 423 =: Kondensatableitung

Die Reaktoren (111), (211) und (311) sind durch eine Vielzahl von Siebböden in einzelne Reaktionskammern unterteilt. Der Abstand der Böden voneinander beträgt das 1 - bis 5-fache, vorzugsweise das 1 - bis 3-fache des Säulendurchmessers.
In der Figur sind die Siebböden lediglich angedeutet.

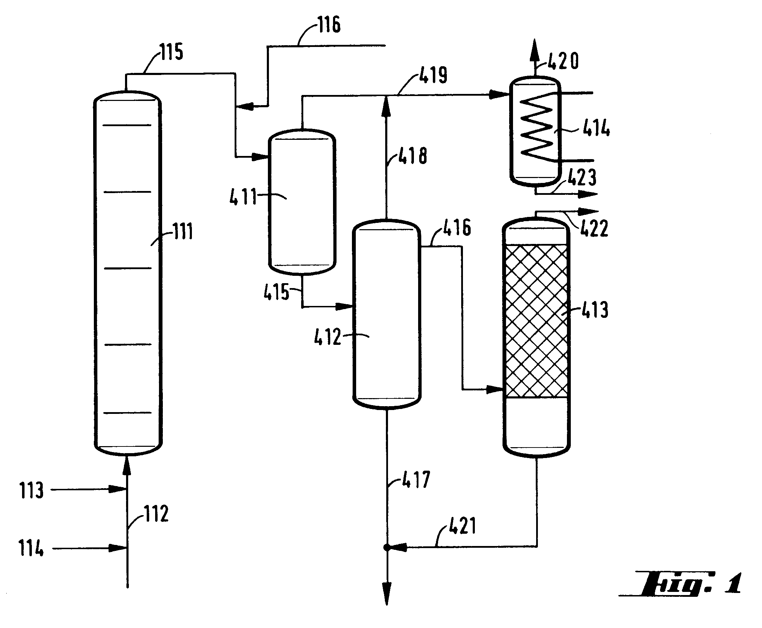

Gemäß Figur 1 ist der Reaktor (111) am Boden mit Zuleitungen für Olefin (112), Chromschwefelsäure (113) und Luft (114) versehen. Vom Kopf des Reaktors (111) führt eine Rohrleitung (115) in das obere Drittel des Entgasungsgefäßes (411). Vom Boden des Entgasungsgefäßes (411) führt eine Rohrleitung in halber Höhe in das Trenngefäß (412), dessen oberer Teil durch eine Rohrleitung (416) mit dem unteren Teil des Entchromers (413) verbunden ist. Vom Kopf des Entgasungsgefäßes (411) führt eine Abgasleitung (419), vom Kopf des Trenngefäßes (412) eine Abgasleitung (418) zu einem Kondensatabscheider (414). Weiterhin führt vom Boden des Trenngefäßes (412) eine Ableitung (417) zu einer (nicht gezeigten) Chromsäure-Aufbereitungsanlage. In diese Ableitung (417) mündet eine vom Boden des Entchromers (413) kommende Ableitung (421). Der Entchromer (413) ist hier als Adsorptionskolonne dargestellt. Er wird über Kopf durch die Leitung (422) entleert. Der Kondensatabscheider (414) ist mit einer Abgasleitung (420) am Kopf und mit einer Ableitung (423) am Boden versehen.

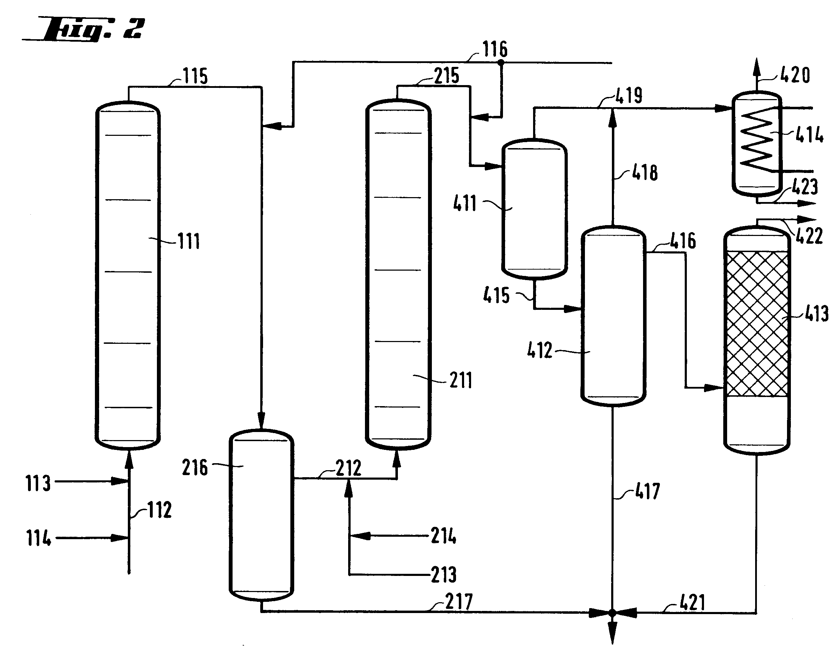

Die Apparatur für das zweistufige Verfahren ist identisch mit der Apparatur für das einstufige Verfahren, jedoch mit den zusätzlichen Apparateteilen der Positionen (211) bis (216). In Figur 2 ist der Reaktor (111) am Boden mit Zuleitungen für Olefin (112), Chromschwefelsäure (113) und Luft (114) versehen. Vom Kopf des Reaktors (111) führt eine Rohrleitung (115) in das Trenngefäß (216), von dessen oberen Drittel eine Rohrleitung (212) zum Boden des Reaktors (211) führt. Gleichzeitig sind eine Zuleitung (214) für Luft und eine Zuleitung (213) für frische Chromschwefelsäure in der Nähe des Bodens des Reaktors (211) an das Rohr (212) angeschlossen. Vom Kopf des Reaktors (211) führt eine Rohrleitung (215) in das obere Drittel des Entgasungsgefäßes (411). Vom Boden des Entgasungsgefäßes (411) führt eine Rohrleitung in halber Höhe in das Trenngefäß (412), dessen oberer Teil durch eine Rohrleitung (416) mit dem unteren Teil des Entchromers (413) verbunden ist. Vom Kopf des Entgasungsgefäßes (411) führt eine Abgasleitung (419), vom Kopf des Trenngefäßes (412) eine Abgasleitung (418) zu einem Kondensatabscheider (414). Weiterhin führt vom Boden des Trenngefäßes (412) eine Ableitung (417) zu einer (nicht gezeigten) Chromsäure-Aufbereitungsanlage. In diese Ableitung (417) mündet eine vom Boden des Entchromers (413) kommende Ableitung (421) und eine Ableitung (217) vom Boden des Trenngefäßes (216).

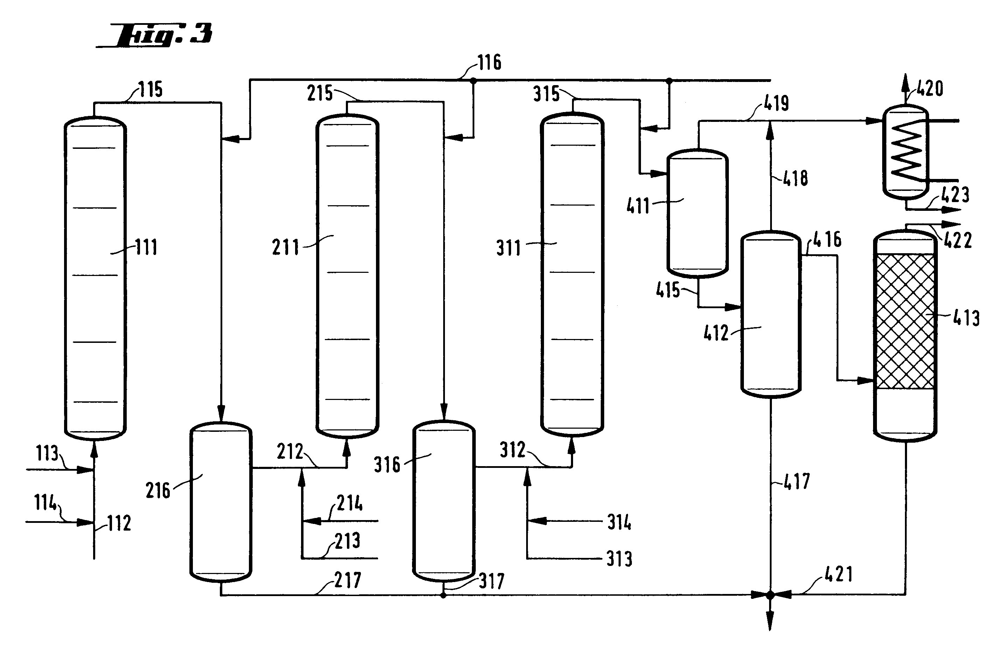

Die Apparatur für eine dreistufige Arbeitsweise gemäß Figur 3 enthält eine weitere Gruppe von Apparateteilen der Positionen (311) bis (316). In Figur 3 ist der Reaktor (111) am Boden mit Zuleitungen für Olefin (112), Chromschwefelsäure (113) und Luft (114) versehen. Vom Kopf des Reaktors (111) führt eine Rohrleitung (115) in das Trenngefäß (216), von dessen oberen Drittel eine Rohrleitung (212) zum Boden des Reaktors (211) führt. Gleichzeitig sind eine Zuleitung (214) für Luft und eine Zuleitung (213) für frische Chromschwefelsäure in der Nähe des Bodens des Reaktors (211) an das Rohr (212) angeschlossen. Vom Kopf des Reaktors (211) führt eine Rohrleitung (215) in das Trenngefäß (316), von dessen oberen Drittel eine Rohrleitung (312) zum Boden des Reaktors (311) führt. Gleichzeitig sind eine Zuleitung (314) für Luft und eine Zuleitung (313) für frische Chromschwefelsäure in der Nähe des Bodens des Reaktors (311) an das Rohr (312) angeschlossen. Vom Kopf des Reaktors (311) führt eine Rohrleitung (315) in das obere Drittel des Entgasungsgefäßes (411). Der Boden des Trenngefäßes (316) ist durch eine Ableitung (317) mit der Ableitung (217) verbunden. Vom Boden des Entgasungsgefäßes (411) führt eine Rohrleitung in halber Höhe in das Trenngefäß (412), dessen oberer Teil durch eine Rohrleitung (416) mit dem unteren Teil des Entchromers (413) verbunden ist. Vom Kopf des Entgasungsgefäßes (411) führt eine Abgasleitung (419), vom Kopf des Trenngefäßes (412) eine Abgasleitung (418) zu einem Kondensatabscheider (414). Weiterhin führt vom Boden des Trenngefäßes (412) eine Ableitung (417) zu einer (nicht gezeigten) Chromsäure-Aufbereitungsanlage. In diese Ableitung (417) mündet eine vom Boden des Entchromers (413) kommende Ableitung (421) und eine Ableitung (217) vom Boden des Trenngefäßes (216).

Sämtliche Reaktoren, Gefäße und Rohrleitungen sind mit Temperiereinrichtungen versehen.

Für die Durchführung des erfindungsgemäßen Verfahrens können wahlweise ein, zwei oder drei Stufen eingesetzt werden. Dazu können die verschiedenen Reaktorstufen miteinander kombiniert werden. Außerdem kann für jede Stufe ein einzelner oder mehrere Reaktoren eingesetzt werden.

Bei der einstufigen Arbeitsweise gemäß Figur 1 wird das Olefin über die Zuleitung (112) zusammen mit einem Teil der Chromschwefelsäure (Zuleitung (113)) und Luft (Zuleitung (114)) in den ersten Reaktor (111) eingeführt. Das Gemisch fließt unter intensiver Durchmischung durch den Reaktor (111). Danach wird das Produkt in eine Entgasungsstufe bestehend aus dem Entgasungsgefäß (411) und dem Trenngefäß (412) geführt. Hier werden die flüchtigen Bestandteile über Kopf abgetrennt, und in einem Kondensatabscheider (414) die kondensierbaren Anteile aus dem Abgasstrom entfernt. Das Abgas wird über die Ableitung (420) und das Kondensat über die Ableitung (423) abgeführt. Nach der Trennung von Oxidat und Oxidationsmittel im Trenngefäß (412) wird das Oxidat noch im Entchromer (413) von kolloidal verteilten oder chemisch gebundenen Chromverbindungen befreit. Diese Reinigung kann in Form einer Waschstufe, einer Adsorptionsstufe oder einer Trennung mittels Zentrifuge erfolgen. Das Oxidat wird über die Leitung (422) abgezogen. Die verbrauchte Chromschwefelsäure wird über die Leitungen (417) und (421) zur Regenerierung geführt.

Bei der zweistufigen Arbeitsweise gemäß Figur 2 wird die Reaktionsmischung aus Reaktor (111) in ein Trenngefäß (216) gegeben. Dort trennt sich das Oxidat von der Chromschwefelsäure, die durch die Leitung (217) zur Regenerierung fließt, und wird mit frischer Chromschwefelsäure und Luft über die Leitung (212) in den Reaktor (211) eindosiert. Von diesem Reaktor strömt die Reaktionsmischung weiter in die obenbeschriebenen Aufbereitungsstufen.

Die dreistufige Arbeitsweise gemäß Figur 3 läuft in der gleichen Weise ab, wobei zwischen den Reaktor (211) und dem Entgasungsgefäß (411) noch ein Reaktor (311) und ein Trenngefäß (316) eingeschaltet sind.

Das Reaktionsgemisch wird somit nach jeder Stufe getrennt und entweder zum Endprodukt aufbereitet oder zusammen mit frischer Chromschwefelsäure und Luft in die nächste Stufe eindosiert.

Im erfindungsgemäßen Verfahren werden Olefine mit interner Doppelbindung mit insgesamt 16 bis 70, vorzugsweise 20 bis 50 C-Atomen eingesetzt. Beispiele für derartige Olefine sind solche der Formel R¹-CH=CH-R², worin R¹ und R² ein Wasserstoffatom oder eine C₁-C₆₈-, vorzugsweise C₁-C₄₈-Alkylgruppe bedeuten und R¹ und R² zusammen 14 bis 68, vorzugsweise 18 bis 48 C-Atome aufweisen, oder der Formel R³-C(R⁴)=CH₂, worin R³ und R⁴ eine C₁-C₆₈-, vorzugsweise C₁-C₄₈-Alkylgruppe bedeuten und R³ und R⁴ zusammen 14 bis 68, vorzugsweise 18 bis 48 C-Atome aufweisen. Beispiele für derartige Olefine sind Hexadecen-1, Octadecen-1, Eicosen-1, Docosen-1, Tetracosen-1, Triaconten-1, 2-Ethyl-triaconten-1 sowie 1-Olefingemische, die vorwiegend aus C₂₂- bis C₅₀-Olefinen bestehen.

Als Oxidationsmittel dient eine Lösung von CrO₃ und Cr₂(SO₄)₃, gegebenenfalls auch von Alkalidichromat, in wäßriger Schwefelsäure. Man verwendet ca. 1-molare Lösungen, bezogen auf den Gehalt an CrO₃. Im allgemeinen enthält die eingesetzte Chromschwefelsäure 500 bis 600 g H₂SO₄ und 95 bis 110 g CrO₃ im dm³.

Die zur Oxidation des Olefins bis zum gewünschten Umsetzungsgrad erforderliche Menge an Chromschwefelsäure kann beim mehrstufigen Verfahren zusammen mit der Olefinschmelze bereits dem Reaktor der ersten Reaktionsstufe zugeführt werden. Besser bewährt hat sich aber die Verfahrensweise, die Chromschwefelsäure in Teilmengen zu dosieren. Vor der Zugabe der frischen Chromschwefelsäure wird dabei das verbrauchte Oxidationsmittel in einem Trenngefäß abgetrennt und ausgeschleust.

Das Mengenverhältnis von Olefin zu Oxidationsmittel hängt dabei von der Molmasse der olefinischen Komponente und deren Reaktivität ab. Bei der Oxidation von einer Olefinmischung aus Vinylolefin, Vinylidenolefin und Olefin mit internen Doppelbindungen mit der Jodzahl (JZ) 45, wie es beispielsweise als ®Chevron C₃₀₊-Olefin auf dem Markt angeboten wird, wird eine Gesamtmenge von 120 bis 150 Gew%, entsprechend 165 bis 200 mol%, an CrO₃ bezogen auf das eingesetzte Olefin benötigt, um zu einer Mischung langkettiger aliphatischer Carbonsäuren mit einer Säurezahl (SZ) von 105 bis 125 zu kommen. In einer zweistufigen Reaktion kann man dabei ca. 90% des eingesetzten Olefins umsetzen. Man erhält ein Carbonsäuregemisch mit SZ 105. Das Oxidationsmittel wird dabei in der ersten Stufe und in der zweiten Stufe mit ca. 60 Gew.-% zugesetzt.
Eine dreistufige Reaktion mit einem Oxidationsmitteleinsatz von 60 Gew.-% in der ersten Stufe, 50 Gew% in der zweiten Stufe und 30% in der dritten Stufe führt bei einem Olefinumsatz von > 95% zu einer Carbonsäuremischung mit einer Säurezahl SZ von 120 bis 125.

Die für das gute Gelingen des Verfahrens notwendige Durchmischung ergibt sich automatisch durch die Art des Reaktors und durch die Wirkung des entstehenden Wasserdampfs und der flüchtigen Reaktionsgase. Zusätzlich wird Luft in den jeweils ersten Reaktor einer Stufe eingeblasen.

Die Reaktion wird bei einer Temperatur von ca. 90°C bis 200°C durchgeführt, bevorzugt im Bereich von 110°C bis 125°C und bei einem Druck von 100 mbar bis 20 bar, bevorzugt 1 bar bis 5 bar. Die Verweilzeiten in den einzelnen Reaktorstufen betragen 60 Minuten bis 180 Minuten. Die Verweilzeit hängt vom Phasenverhältnis und vom angestrebten Olefin- bzw. Oxidationsmittelumsatz ab.

Carbonsäuremischungen, hergestellt nach dem erfindungsgemäßen Verfahren, können mit Monoalkoholen oder Polyolen wie Glykol, Glycerin, Trimethylolpropan, Pentaerythrit oder Sorbitol zu Estern und Partialestern oder zu Mischungen solcher Ester verarbeitet wurden. Durch Umsetzung mit basischen Oxiden oder Metallhydroxiden können durch direkte Umsetzung oder durch Umfällung die entsprechenden Seifen der Carbonsäuren hergestellt werden. Durch Umsetzung mit Aminen oder Aminoalkoholen gelangt man zu den Amiden , Amidestern und Aminoestern.

Diese Produkte zeichnen sich durch eine helle Farbe, gute Thermostabilität und ein breites Anwendungsspektrum aus.

So können die Ester und/oder Seifen als Pastenwachse, Emulsionswachse, Trennmittel oder als Gleitmittel eingesetzt wurden.

Die nachstehenden Beispiele sollen das Verfahren näher erläutern.

Für die Versuche wurde eine Laborapparatur mit ca. 6 dm³ Reaktorinhalt pro Reaktionsstufe eingesetzt.

### Beispiel 1

### Einstufige Oxidation mit variablem Oxidationsmittelanteil

Eine Olefinmischung aus Vinylolefin, Vinylidenolefin und Olefin mit internen Doppelbindungen mit einer Jodzahl (JZ) von 45 (Chevron C₃₀₊-Olefin) wurde aufgeschmolzen und in ein beheiztes Vorratsgefäß gegeben. Die Temperatur der Schmelze wurde durch Dampfheizung auf 95 bis 100°C eingestellt. Über eine dampfbeheizte Leitung und eine beheizte Pumpe wurde das Olefin in den ersten Reaktor gefördert.

Die Chromsäure wurde aus einem temperierten Vorratsgefäß über eine säurefeste Pumpe in den Reaktor gefördert. Luft wurde mit einem Druck von 0,5 bar in einer Menge von 100 dm³/h in den Reaktor eingeleitet. Die drei Komponenten wurden am Reaktorboden in einer Mehrstoffdüse gemischt und in die erste Reaktorkammer eingespritzt. Die Reaktionsmischung wurde durch die Reaktorkammern gefördert und nach Durchlaufen der einzelnen Reaktorstufen über die Entgasung in das Trenngefäß geführt. Dort wurde das verbrauchte Oxidationsmittel abgetrennt. An dieser Stelle konnte durch Zugabe eines Trennmittels (Menge 0,1 bis 0,5 Gew.-%, bezogen auf eingesetztes Olefin) die Trennung verbessert werden.

Umsatz an Olefin und Oxidationsmittel hängen dabei von der Fördermenge und damit der Verweilzeit im Reaktor ab.

Das so erhaltene Oxidat wurde noch durch Zentrifugieren von restlichen Chromverbindungen befreit.

**Tabelle 1**

| Kontinuierliche Oxidation von α-Olefin Versuche mit konstanter Olefin/Chromsäure-Dosierung | | | | | |
|---|---|---|---|---|---|
| Versuch | Wachs cm³/h | Chroms. cm³/h | CrO₃ E % | SZ | CrO₃ V % |
| 1 | 400 | 3000 | 75 | 62 | 60 |
| 2 | 600 | 3000 | 50 | 56 | 48 |
| 3 | 600 | 3600 | 60 | 61 | 50 |
| 4 | 400 | 3600 | 90 | 74 | 72 |
| 5 | 650 | 3600 | 55 | 58 | 52 |
| 6 | 650 | 3600 | 55 | 50 | 43 |
| 7 | 550 | 3000 | 55 | 58 | 48 |
| E = Einsatz in Gew-%, bezogen auf Olefin V = Verbrauch in Gew-%, bezogen auf Olefin | | | | | |

### Beispiel 2

### Einstufiges Verfahren mit variabler Dosiermenge bei konstantem Oxidationsmittelanteil

Das Olefin wurde aufgeschmolzen und in ein beheiztes Vorratsgefäß gegeben. Die Temperatur der Schmelze wurde durch Dampfheizung auf 95 bis 100°C eingestellt. Über eine dampfbeheizte Leitung und eine beheizte Pumpe wurde das Olefin in den ersten Reaktor gefördert. Die Chromsäure wurde aus einem temperierten Vorratsgefäß über eine säurefeste Pumpe in den Reaktor gefördert. Luft wurde mit einem Druck von 0,5 bar in einer Menge von 100 dm³/h in den Reaktor eingeleitet.

Die drei Komponenten wurden am Reaktorboden in einer Mehrstoffdüse gemischt und in die erste Reaktorkammer eingespritzt. Bei der Befüllung der Reaktoren konnte mit einer erhöhten Förderleistung gearbeitet werden. Zur Einstellung einer optimalen Oxidatsmittelnutzung wurde die Fördermenge von Olefin und Chromsäure dann reduziert.

Die Reaktionsmischung wurde durch die Reaktorkammern gefördert und nach Durchlaufen der einzelnen Reaktorstufen über die Entgasung in das Trenngefäß geführt. Dort wurde das verbrauchte Oxidationsmittel abgetrennt. An dieser Stelle konnte durch Zugabe eines Trennmittels (Menge 0,1 bis 0,5 Gew.-%, bezogen auf eingesetztes Olefin) die Trennung verbessert werden.

Das so erhaltene Oxidat wurde noch durch Zentrifugieren von Restchrom befreit.

**Tabelle 2**

| Kontinuierliche Oxidation von α-Olefin Versuche mit variabler Olefin/Chromsäure-Dosierung | | | | | |
|---|---|---|---|---|---|
| Versuch | Wachs 10 cm³/h | Chroms. 100 cm³/h | CrO₃ E % | SZ | CrO₃ V % |
| 11 | 65-50-40 | 39-30-24 | 60 | 57 | 56 |
| 13 | 65 - 40 | 39 - 24 | 60 | 65 | 59 |
| 15 | 60 - 40 | 36 - 24 | 60 | 57 | 57 |
| 17 | 60 - 40 | 36 - 24 | 60 | 58 | 58 |
| E = Einsatz in Gew.-%, bezogen auf Olefin V = Verbrauch in Gew.-%, bezogen auf Olefin | | | | | |

### Beispiel 3

### Zweistufiges Verfahren mit konstantem Oxidationsmittelanteil und variabler Dosierung

Das Olefin wurde aufgeschmolzen und in ein beheiztes Vorratsgefäß gegeben. Die Temperatur der Schmelze wurde durch Dampfheizung auf 95 bis 100°C eingestellt. Über eine dampfbeheizte Leitung und eine beheizte Pumpe wurde das Olefin in den ersten Reaktor gefördert. Die Chromsäure wurde aus einem temperierten Vorratsgefäß über eine säurefeste Pumpe in den Reaktor gefördert. Luft wurde mit einem Druck von 0,5 bar in einer Menge von 100 dm³/h in den Reaktor eingeleitet.

Die drei Komponenten wurden am Reaktorboden in einer Mehrstoffdüse gemischt und in die erste Reaktorkammer eingespritzt. Die Reaktionsmischung wurde durch die Reaktorkammern gefördert und nach Durchlaufen der ersten Reaktionsstufe in das Trenngefäß gegeben, das verbrauchte Oxidationsmittel wurde abgetrennt und das voroxidierte Olefin wurde zusammen mit frischer Chromsäure und Luft in die zweite Reaktionsstufe gefördert. Nach dem Durchlaufen aller Reaktorkammern wurde das Produkt entgast, das verbrauchte Oxidationsmittel wurde abgetrennt und in einer Zentrifuge wurden aus dem Oxidat restliche Chromverbindungen abgetrennt.

**Tabelle 3**

| Kontinuierliche Oxidation von α-Olefin Versuche mit variabler Olefin/Chromsäure-Dosierung, 2. Stufe | | | | | |
|---|---|---|---|---|---|
| Versuch | Wachs 10 cm³/h | Chroms. 100 cm³/h | CrO₃ E % | SZ | CrO₃ V % |
| 10 (1 - 5) | 60 - 50 | 36 - 30 | 60 | 109 | 54 |
| 8 ( 6 ) | 65 - 45 | 36 - 25 | 55 | 101 | 55 |
| 9 ( 7 ) | 60 - 40 | 36 - 24 | 60 | 108 | 60 |
| 12 ( 11 ) | 65 - 40 | 39 - 24 | 60 | 108 | 59 |
| 14 ( 13 ) | 65 - 40 | 39 - 24 | 60 | 110 | 58 |
| 18 ( 17 ) | 60 - 40 | 36 - 24 | 60 | 109 | 58 |
| E = Einsatz in Gew.-%, bezogen auf Olefin V = Verbrauch in Gew.-%, bezogen auf Olefin Die Werte in () geben die Versuchsnummer der ersten Stufe an | | | | | |

### Beispiel 4

### Dreistufiges Verfahren mit konstantem Oxidationsmittelanteil und variablen Dosiermengen

Man verfuhr wie in Beispiel 3. Nach der zweiten Reaktionsstufe wurde das Oxidat mit frischer Chromsäure in die dritte Reaktionsstufe eingeführt und wie oben beschrieben aufgearbeitet. Man erhielt beim Einsatz von 50 Gew% CrO₃ ein Oxidat mit SZ 125.

## Patentansprüche

1. Verfahren zur kontinuierlichen Herstellung von höhermolekularen, linearen aliphatischen Carbonsäuren durch Oxidation von Olefinen mit 16 bis 70 C-Atomen mit ca. 1-molarer, bezogen auf CrO₃, Chromschwefelsäure bei einer Temperatur von 90 bis 200°C und einem Druck von 100 mbar bis 20 bar,
dadurch gekennzeichnet,
daß die Reaktion im Gleichstrom in mindestens einem Reaktor mit engem Verweilzeitspektrum durchgeführt wird, wobei die Durchmischung der Reaktanden durch den infolge der Reaktionswärme entstehenden Wasserdampf und das Reaktionsgas sowie zusätzliches Einblasen von Luft erfolgt.

2. Verfahren nach Anspruch 1, dadurch gekennzeichnet, daß die Reaktion in einem kaskadierten Blasensäulenreaktor durchgeführt wird.

3. Verfahren nach Anspruch 1, dadurch gekennzeichnet, daß die Reaktion mehrstufig durchgeführt wird, wobei verbrauchte Chromschwefelsäure nach jeder Stufe abgetrennt wird und die für die Oxidation notwendige Menge an Chromschwefelsäure kontinuierlich in Teilmengen zu Beginn jeder Stufe zudosiert wird.

4. Verfahren nach Anspruch 1, dadurch gekennzeichnet, daß C₂₀-C₅₀ Olefine mit internen Doppelbindungen oder mit Vinylidendoppelbindungen oder mit Vinyldoppelbindungen oder Mischungen dieser Verbindungen eingesetzt werden.

5. Verfahren nach Anspruch 1, dadurch gekennzeichnet, daß die Trennung zwischen organischer Phase und anorganischer Phase im Trenngefäß durch die Zugabe grenzflächenaktiver Verbindungen beschleunigt wird.

## Claims

1. A process for the continuous preparation of relatively high-molecular-weight, linear aliphatic carboxylic acids by oxidation of olefins having from 16 to 70 carbon atoms with approximately 1 molar, based on CrO₃, chromic/sulfuric acid at a temperature of from 90 to 200°C and a pressure of from 100 mbar to 20 bar, which comprises carrying out the reaction in cocurrent mode in at least one reactor having a narrow residence time spectrum, the mixing of the reactants being carried out by the steam formed as a result of the heat of reaction and the reaction gas and also additional blowing-in of air.

2. The process as claimed in claim 1, wherein the reaction is carried out in a cascaded bubble column reactor.

3. The process as claimed in claim 1, wherein the reaction is carried out in a plurality of stages, used chromic/sulfuric acid being separated off after each stage and the amount of chromic/sulfuric acid required for the oxidation being continuously metered in in portions at the beginning of each stage.

4. The process as claimed in claim 1, wherein C₂₀-C₅₀ olefins having internal double bonds or having vinylidene double bonds or having vinyl double bonds or mixtures of these compounds are used.

5. The process as claimed in claim 1, wherein the separation of the organic phase and the inorganic phase in the separation vessel is accelerated by the addition of surface-active compounds.

## Revendications

1. Procédé de préparation en continu d'acides carboxyliques aliphatiques linéaires de haut poids moléculaire, par oxydation d'oléfines contenant 16 à 70 atomes de carbone avec un mélange sulfochromique 1 molaire environ par rapport à CrO₃, à une température de 90 à 200°C et une pression de 100 mbar à 20 bar, caractérisé en ce que la réaction est réalisée à courants parallèles, dans au moins un réacteur à intervalle étroit de temps de séjour, le mélange intime des réactants se faisant à l'aide de la vapeur d'eau qui se forme suite à la chaleur réactionnelle et du gaz de réaction ainsi que par une insufflation supplémentaire d'air.

2. Procédé selon la revendication 1, caractérisé en ce que la réaction est réalisée dans un réacteur à colonnes à bulles en cascade.

3. Procédé selon la revendication 1, caractérisé en ce que la réaction est réalisée en plusieurs étapes, le mélange sulfochromique usé étant séparé après chaque étape et la quantité de mélange sulfochromique nécessaire pour l'oxydation étant ajoutée en continu par quantités partielles au début de chaque étape.

4. Procédé selon la revendication 1, caractérisé en ce que l'on utilise des oléfines C₂₀ à C₅₀ à doubles liaisons internes ou à doubles liaisons vinylidèniques ou à doubles liaisons vinyliques ou des mélanges de ces composés.

5. Procédé selon la revendication 1, caractérisé en ce que la séparation entre phase organique et phase inorganique dans le récipient de séparation est accélérée par l'ajout de composés tensioactifs.
